# EUROPEAN PATENT APPLICATION

(11) **EP 3 264 300 A1**
(43) Date of publication of application: **03.01.2018**
(21) Application number: 17176215.6
(22) Date of filing: 15.06.2017
(51) Int. Cl.: G06F 19/00

(54) **PATIENT INFORMATION DISPLAY SYSTEM AND PATIENT INFORMATION DISPLAY METHOD**

(30) Priority: 23.06.2016 JP 2016124031
(71) Applicant: Konica Minolta, Inc., Tokyo 100-7015 (JP)
(72) Inventor: MOTOKI, Wataru, Chiyoda-ku, Tokyo 100-7015 (JP); TAKESUE, Kanako, Chiyoda-ku, Tokyo 100-7015 (JP)
(74) Representative: Atkinson, Ian Anthony

(57) **Abstract**

A patient information display system includes a storage for storing patient information on a patient in a classified manner as reference information which indicates a condition of the patient at a predetermined time point or time-series information which is generated after the predetermined time point and is accumulated as time proceeds. Each of the patient information is stored in the storage along with an attribute of a user who registered the patient information. The patient information display system further includes a hardware processor which retrieves patient information of a target patient and an attribute linked to the patient information from the storage and displays the patient information of the target patient in a single screen on a display in which the screen is divided into respective areas for reference information and time-series information with respect to each attribute.

## Description

### BACKGROUND OF THE INVENTION

### 1. Field of the Invention

The present invention relates to a patient information display system and a patient information display method.

### 2. Description of Related Art

In the medical field, home care has recently become more important. Since nursing and care of a home-care patient requires 24-hour 365-day support, cooperation between clinics, home-visit nursing care service and long-term care service, patient families and the like is important.

Further, the past medical history and the treatment history of a patient sometimes has a great influence on the future treatment. In view of them, a technique has been proposed which involves receiving an input of specifying a predetermined period in a timeline displayed on the display apparatus, searching electronic medical record information on medical examinations of a patient in the specified period, extracting the examined body parts from the electronic medical record information, and overlaying marks on an outer appearance image of the patient at the positions of the examined body parts (see JP 2012-247879A and JP 2012-247880A).

In order to improve the care of a home care patient, it is required to check both reference information (baseline information) that represents the patient condition at the time he/she started to be cared at home and time-series information (timeline information) that is generated after he/she started to be cared at home. In home care, it is also important who with which attribute provided each information since many persons with different relationship with the patient such as physicians, nurses, caregivers and patient family members are involved.

There has been no screen structure which enables displaying the patient information on a patient in a single screen in a classified manner as reference information or time-series information along with indication of the source of each information.

### SUMMARY OF THE INVENTION

The present invention has been made in view of the above-described problem with the prior art, and an object thereof is to display patient information in an easy-to-understand manner.

In order to realize the above object, according to a first aspect of the present invention, there is provided a patient information display system, including:
a storage for storing patient information on a patient in a classified manner as reference information which indicates a condition of the patient at a predetermined time point or time-series information which is generated after the predetermined time point and is accumulated as time proceeds,
wherein each of the patient information is stored in the storage along with an attribute of a user who registered the patient information,
wherein the patient information display system further includes: a hardware processor which retrieves patient information of a target patient and an attribute linked to the patient information from the storage and displays the patient information of the target patient in a single screen on a display in which the screen is divided into respective areas for reference information and time-series information with respect to each attribute.

According to a second aspect of the present invention, there is provided a patient information display method for a patient information display system that includes a storage for storing patient information on a patient in a classified manner as reference information which indicates a condition of the patient at a predetermined time point or time-series information which is generated after the predetermined time point and is accumulated as time proceeds,
wherein each of the patient information is stored in the storage along with an attribute of a user who registered the patient information,
the method including:
retrieving patient information of a target patient and an attribute linked to the patient information from the storage; and
displaying the patient information of the target patient in a single screen on the display in which the screen is divided into respective areas for reference information and time-series information with respect to each attribute.

### BRIEF DESCRIPTION OF THE DRAWINGS

The present invention will become more fully understood from the detailed description given hereinbelow and the appended drawings which are given by way of illustration only, and thus are not intended as a definition of the limits of the present invention, and wherein:
FIG. 1 is a system configuration view of a home care cooperation system according to an embodiment of the present invention;
FIG. 2 is a block diagram of the functional configuration of a data management apparatus;
FIG. 3 illustrates an example of a user management table;
FIG. 4 illustrates an example of a patient information table;
FIG. 5 illustrates an example of a basic information table;
FIG. 6 illustrates an example of a baseline table;
FIG. 7 illustrates an example of a timeline table;
FIG. 8 illustrates an example of an access restriction table;
FIG. 9 illustrates the folder structure of a file storage;
FIG. 10 illustrates an example of patient information;
FIG. 11 is a block diagram of the functional configuration of a user terminal;
FIG. 12 is a ladder chart of a patient registration processing that is performed by a data management apparatus and a user terminal;
FIG. 13 is a ladder chart of a patient information upload processing that is performed by the data management apparatus and the user terminal;
FIG. 14 illustrates an example of a patient list window;
FIG. 15 illustrates an example of a file management window;
FIG. 16 illustrates an example of an edit window;
FIG. 17 is a ladder chart of a patient information reference processing that is performed by the data management apparatus and the user terminal;
FIG. 18 illustrates an example of a patient information reference window;
FIG. 19 illustrates an example of a patient basic information window; and
FIG. 20 illustrates an example of a vital sign window.

### PREFERRED EMBODIMENT OF THE INVENTION

Hereinafter, an embodiment of the patient information display system according to the present invention will be described referring to the drawings. However, it is not intended that the scope of the present invention is limited to the illustrated examples.

### CONFIGURATION OF HOME CARE COOPERATION SYSTEM

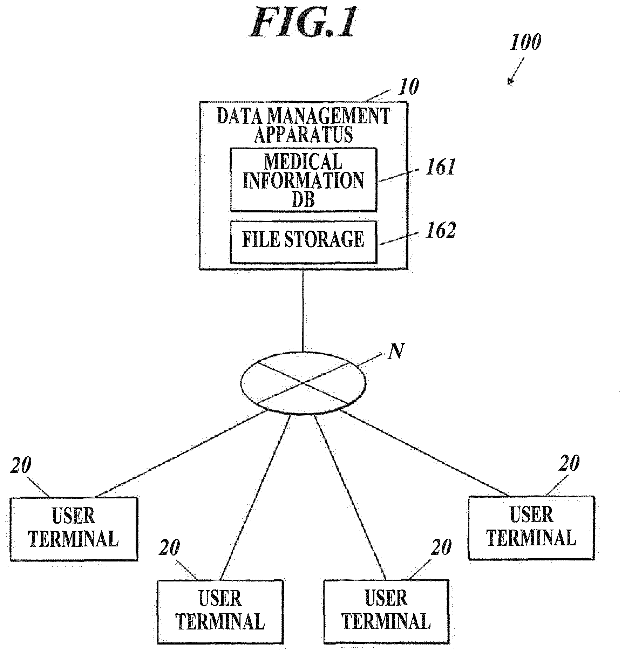

FIG. 1 illustrates the system configuration of a home care cooperation system 100 as the patient information display system according to an embodiment.

As illustrated in FIG. 1, the home care cooperation system 100 includes a data management apparatus 10 that is installed in a regional data center and user terminals 20 that can be used by users such as a home-care patient, family members of the patient and physicians, nurses, caregivers and the like belonging to facilities for medical care, nursing and care who are involved in the home care. The data management apparatus 10 are connected to the user terminal 20 through a communication network N.

The data management apparatus 10, which includes a medical information DB (Data Base) 161, a file storage 162 and the like, accumulates and manages medical information that is registered on the user terminals 20 by the users. The data management apparatus 10 may be constituted by a cloud server provided in a cloud environment.

The user terminals 20 are constituted by PCs (Personal Computers) tablet terminals, smartphones and the like that are used in facilities and the home of a home-care patient. Such facilities include the hospital or clinic of the primary care physician of a patient, home-visit nursing care services of the nurses who provide home-visit nursing to a home-care patient, care coordinators who develop a care plan of a home-care patient, long-term care services of caregivers who provide home care to a home-care patient.

The users involved in the home care of a home-care patient register patient information on the patient through the user terminals 20. The registered patient information is stored in the data management apparatus 10 and is disclosed to the members of the team for a home-care patient.

### CONFIGURATION OF DATA MANAGEMENT APPARATUS

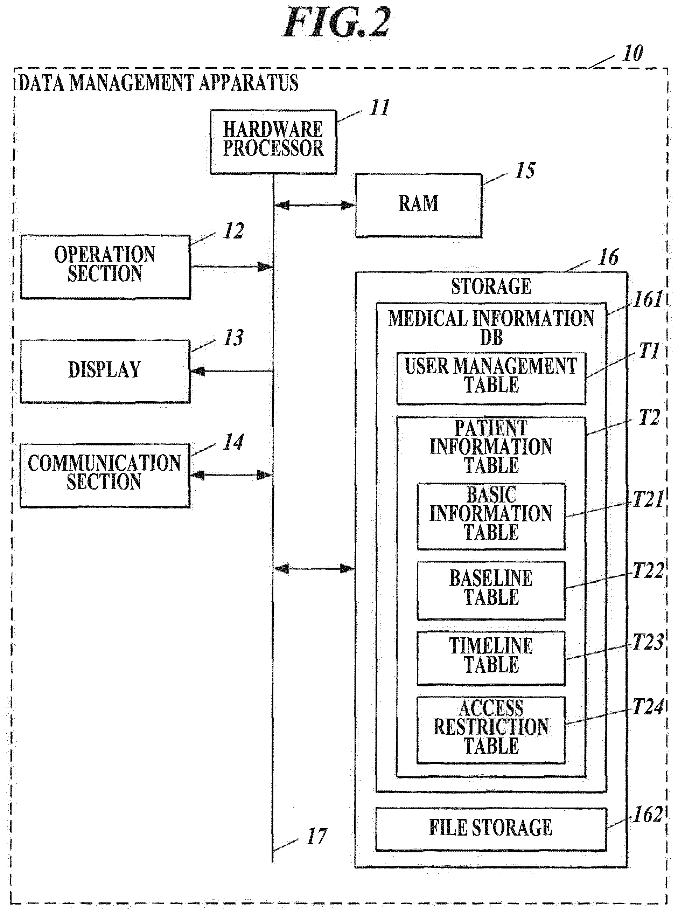

FIG. 2 illustrates the functional configuration of the data management apparatus 10.

As illustrated in FIG. 2, the data management apparatus 10 includes a hardware processor 11, an operation section 12, a display 13, a communication section 14, a RAM 15, a storage 16 and the like, which are connected to each other through a bus 17.

The hardware processor 11, which is constituted by a CPU (Central Processing Unit) and the like, integrally controls the processing operation of the components of the data management apparatus 10. Specifically, the CPU reads out various processing programs stored in the storage 16, develops them in the RAM 15 and performs a variety of processing in cooperation with the various processing programs according to operation signals input from the operation section 12 or order signals received from the communication section 14.

The operation section 12, which includes a keyboard with cursor keys, character input keys, a variety of function keys and the like, and a pointing device such as a mouse, outputs an operation signal input by means of a key operation on the keyboard or a mouse operation to the hardware processor 11. Alternatively, the operation section 12 may be constituted by a touch panel laminated on the display 13 and outputs an operation signal corresponding to the position of a touch gesture with a finger or the like of the operator to the hardware processor 11.

The display 13, which includes a monitor such as an LCD (Liquid Cristal Display) or the like, displays a variety of windows according to a display signal input from the hardware processor 11.

The communication section 14, which is constituted by a network interface and the like, sends and receives data to and from an external device that is connected through a communication network N such as a LAN (Local Area Network), a WAN (Wide Area Network) or the Internet.

The RAM 15 forms a work area for temporarily storing various programs read out from the storage 16, input or output data and parameters in the variety of processing that is performed and controlled by the hardware processor 11.

The storage 16 is constituted by an HDD (Hard Disk Drive), a non-volatile semiconductor memory and the like. In the storage 16, various processing programs, parameters and files necessary for executing the programs and the like are stored. For example, a web server program for providing a function as a web server that communicates with a web browser installed in the user terminals 20 in the HTTP protocol to provide a variety of web pages to the web browser, an application program that runs on the web server for providing a home care cooperation service to the users of the user terminals 20 through the web browser, and the like are stored in the storage 16. The home care cooperation service refers to a service for sharing information between the team for a home-care patient.

In the storage 16, patient information on a patient is stored in a classified manner as reference information (baseline information) that represents the condition of the patient at a predetermined time point or time-series information (timeline information) that is generated after the predetermined time point and is accumulated as time proceeds. In the embodiment, the predetermined time point corresponds to the time when the patient started to be cared at home. For example, the time when a patient started to be cared at home can correspond to the time when an inpatient was discharged to be a home-care patient, the time when an outpatient becomes a home-care patient, or the like.

The reference information includes, for example, a medical certificate, a medical history, opinions on the hospital discharge, a disease name, a prescription and the like.

The time-series information includes vital signs after the start of home care, prescriptions, laboratory tests, photographs and images, a nursing record, a care record, an electronic medical record, an SNS and the like.

The storage 16 includes a medical information DB 161 and a file storage 162.

The medical information DB 161 is provided to store and manage medical information.

The medical information DB 161 includes a user management table T1 and a patient information table T2.

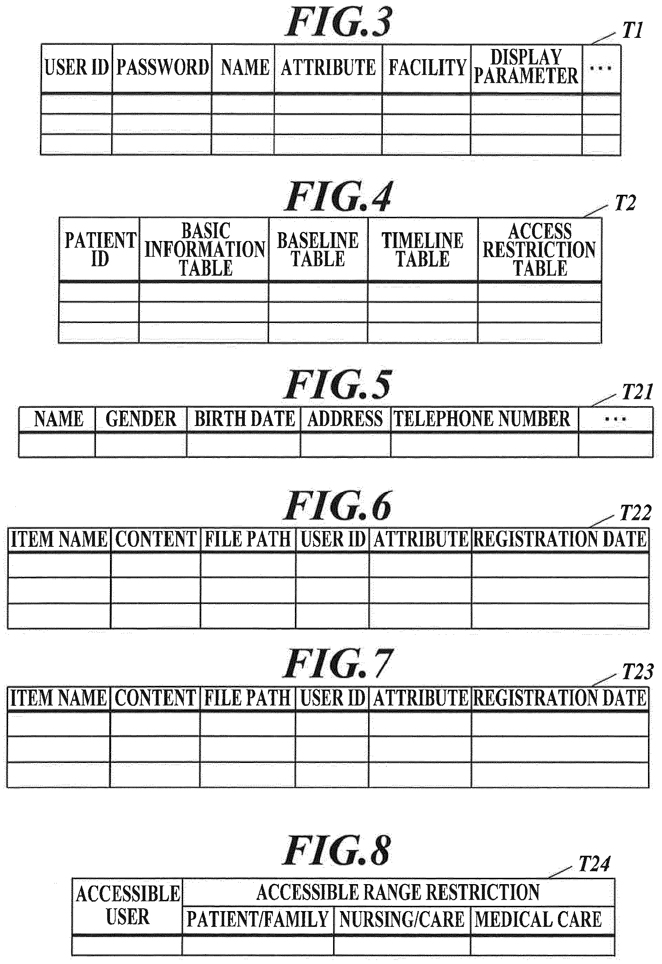

FIG. 3 illustrates an example of the user management table T1. The user management table T1 is provided to manage information on users with respect to each of the users of the home care cooperation system 100. In the user management table T1, a user ID, a password, a name, an attribute, a facility name, a display parameter and the like are stored with a link to each other. That is, the storage 16 functions as a second storage, which stores the user management table T1 that links attributes to user IDs and the names.

The user ID is identification information of a user.

The password is used for user authentication when a user logs in the system.

The name is the name of a user.

The attribute is the attribute of a user. In the embodiment, the attribute is classified into three groups of "patient and family members of the patient (hereinafter referred to as patient/family), "nursing/care" and "medical care".

The facility name is the name of a medical facility to which a user belongs. It is voluntary to register the facility name. For example, when the user is a patient or a family member of a patient, the facility name is null.

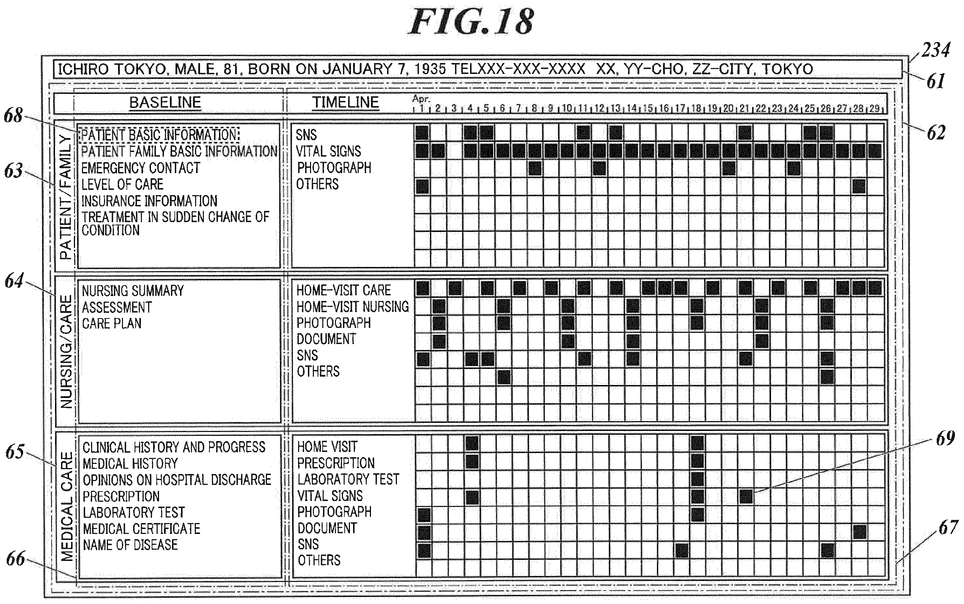

The display parameter is a parameter that determines the display size of each area in a patient information reference window 234 (see FIG. 18).

FIG. 4 illustrates an example of the patient information table T2. The patient information table T2 is provided to manage patient information on patients with respect to each patient. In the patient information table T2, a patient ID, information on a link to a basic information table T21 of the patient with the corresponding patient ID, information on a link to a baseline table T22 of the patient with the corresponding patient ID, information on a link to a timeline table T23 of the patient with the corresponding patient ID and information on a link to an access restriction table T24 of the patient with the corresponding patient ID are stored with a link to each other.

The patient ID is an identification information of a patient.

FIG. 5 illustrates an example of the basic information table T21. The basic information table T21 is provided with respect to each patient to store patient basic information. In the basic information table T21, the name, gender, birth date, address, telephone number and the like of a patient is stored.

FIG. 6 illustrates an example of a baseline table T22. The baseline table T22 is provided with respect to each patient to store reference information of a patient. In the baseline table T22, an item name of reference information, a content, a file path, a user ID of a user who registered the corresponding reference information, an attribute of a user who registered the corresponding reference information and a registration date are stored with a link to each other. When reference information is represented by characters and numbers (hereinafter referred to as character/number information), it is stored in the "content" field. When reference information is in the form of a file (hereinafter referred to as file information), information indicating the storage location of the file in the file storage 162 is stored in the "file path" field.

The patient basic information that is stored in the basic information table T21 may be included in the reference information that is stored in the baseline table T22.

FIG. 7 illustrates an example of the timeline table T23. The timeline table T23 is provided with respect to each patient to store time-series information of a patient. In the timeline table T23, an item name of time-series information, a content, a file path, a user ID of a user who registered corresponding time-series information, an attribute of a user who registered corresponding time-series information, a registration date are stored with a link to each other. When time-series information is character/number information, the content of the time-series information is stored in the "content" field. When time-series information is file information, information indicating the storage location of the file in the file storage 162 is stored in the "file path" field.

FIG. 8 illustrates an example of the access restriction table T24. The access restriction table T24 is provided with respect to each patient to define access restriction to patient information. The access restriction table T24 includes an "accessible user" field and also includes a "patient/family" field, a "nursing/care field" and a "medical care" field as "accessible range restriction".

In the "accessible user" field, a user ID of a user accessible to the patient information of corresponding patient is stored. Such accessible users may include physicians, nurses, caregivers, patients and family members of patients as members of the team for the home care of a corresponding patient.

The "accessible range restriction" defines the registrant attribute of accessible patient information with respect to each attribute of users (who reference the information).

Specifically, in the "patient/family" field, the registrant attribute (patient/family, nursing/care and/or medical care) of patient information that is accessible for users with an attribute of "patient/family" is specified.

In the "nursing/care" field, the registrant attribute (patient/family, nursing/care and/or medical care) of patient information that is accessible for users whose attribute is "nursing/care" is specified.

In the "medical care" field, the registrant attribute (patient/family, nursing/care and/or medical care) of patient information that is accessible for users whose attribute is "medical care" is specified.

In the access restriction table T24, information (accessible range restriction) that indicates the accessibility to the patient information of a patient (patient ID) who is linked to the access restriction table T24 in the patient information table T2 is defined with respect to each attribute. That is, the storage 16 functions as a third storage, which stores the patient information table T2 and the access restriction table T24.

In the file storage 162, file information of documents, photographs, images (X-ray images, etc.) and the like of patient information is stored.

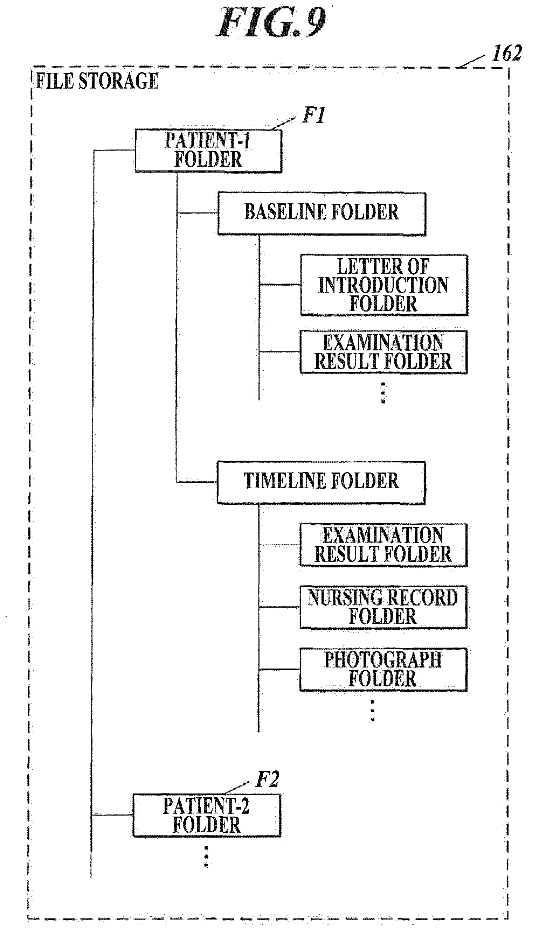

FIG. 9 illustrates the folder structure of the file storage 162.

In the file storage 162, dedicated patient folders F1, F2, ... are created for respective registered patients. In each of the dedicated patient folders F1, F2, ..., a baseline folder and a timeline folder are created. In the baseline folder, patient information that is classified as reference information is stored. In the timeline folder, patient information that is classified as time-series information is stored. In the baseline folder and the timeline folder, folders of respective types of information are created.

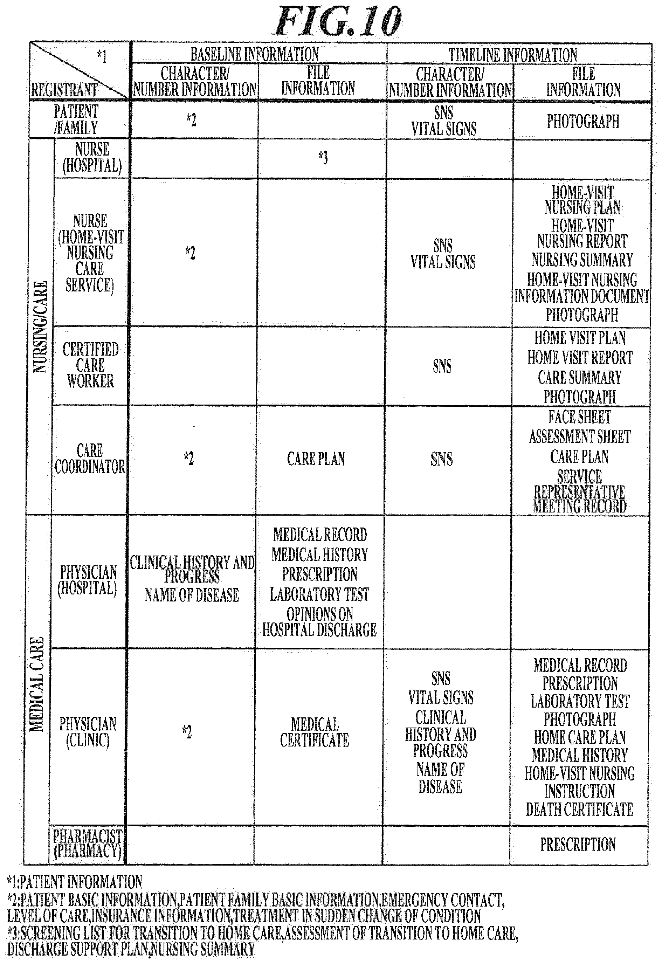

FIG. 10 illustrates examples of possible registrable patient information with respect to each attribute of registrants (users who registers patient information). In FIG. 10, a variety of patient information is classified as reference information (baseline information) or time-series information (timeline information), each of which is further classified as character/number information or file information. In FIG. 10, patient basic information is included in baseline information.

When there is an access to the data management apparatus 10 from a user terminal 20 with a login account (user ID and password) of a certain user, the hardware processor 11 references the user management table T1 in the storage 16 so as to make a determination as to whether the user is a registered user. When the combination of the user ID and the password input on the user terminal 20 is registered in the user management table T1, the hardware processor 11 provides the home care cooperation service of the home care cooperation system 100 to the user having the user ID.

The hardware processor 11 retrieves the patient information of a target patient and the attribute linked to this patient information from the storage 16. Specifically, the hardware processor 11 references the patient information table T2 to specify a baseline table T22 and a timeline table T23 that are linked to the patient ID of the target patient. Then, with regard to the reference information of the target patient, the hardware processor 11 references the specified baseline table T22 to retrieve a content or a file path and an attribute with respect to each item. When it is file information, the hardware processor 11 further retrieves a file from the file storage 162 based on the file path. With regard to time-series information of the target patient, the hardware processor 11 references the specified timeline table T23 to retrieve a content or a file path, an attribute and a registration date with respect to each item. When it is file information, the hardware processor 11 further retrieves a file from the file storage 162 based on the file path.

The hardware processor 11 generates a display data for displaying the patient information of the target patient in a single screen on the display 23 of the user terminal 20 in which the screen is divided into respective areas of the reference information and the time-series information with respect to each attribute.

In this process, the hardware processor 11 retrieves the attribute of the user who currently uses the home care cooperation system 100 from the user management table T1, retrieves information (accessible range restriction) indicating the accessibility to the information of the target patient for users with the retrieved attribute from the access restriction table T24 linked to the patient ID of the target patient, and restricts the contents to be displayed on the display 23 of the user terminal 20 based on the retrieved information indicating the accessibility.

The hardware processor 11 generates a display data for displaying the presence or absence of the time-series information of the target patient along a timeline. Specifically, based on the item names and the registration dates that are retrieved from the timeline table T23 linked to the patient ID of the target patient, the hardware processor 11 puts a mark indicating the presence of time-series information at the position of the registration date on the timeline with respect to each item.

In response to an operation on a user terminal 20 by a user, the hardware processor 11 changes the display size of the areas in the single screen in which the reference information and the time-series information are respectively displayed. That is, the hardware processor 11 functions as a changer.

Further, in response to an operation on a user terminal 20 by a user, the hardware processor 11 changes the display size of the areas for the respective attributes in the single screen. That is, the hardware processor 11 functions as a second changer.

### CONFIGURATION OF USER TERMINAL

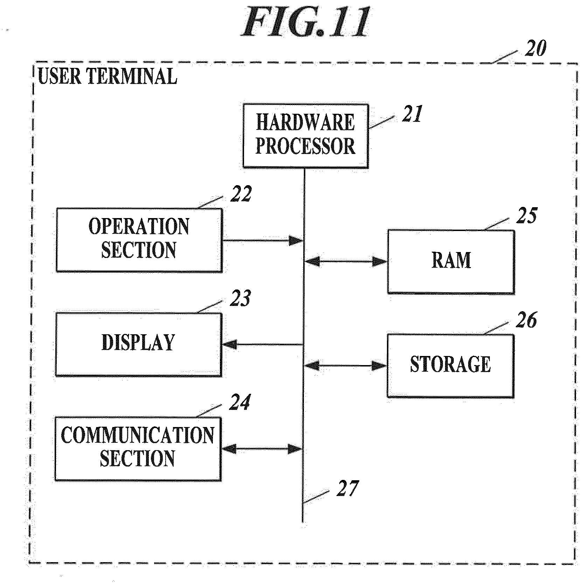

FIG. 11 illustrates the functional configuration of the user terminals 20.

As illustrated in FIG. 11, each of the user terminals 20 includes a hardware processor 21, an operation section 22, a display 23, a communication section 24, a RAM 25, a storage 26 and the like, which are connected to each other through a bus 27.

The hardware processor 21, which is constituted by a CPU and the like, integrally controls the processing operation of the components of the user terminal 20. Specifically, the CPU reads out a variety of processing programs stored in the storage 26, develops them in the RAM 25 and performs a variety of processing in cooperation with the programs according to operation signals input on the operation section 22 and order signals received through the communication section 24.

The operation section 22, which includes a keyboard with cursor keys, character input keys and a variety of function keys, and a pointing device such as a mouse, outputs an operation signal input by means of a key operation on the keyboard or a mouse operation to the hardware processor 21. Alternatively, the operation section 22 may be constituted by a touch panel laminated on the display 23 and outputs an operation signal corresponding to the position of a touch gesture with a finger of the user or the like to the hardware processor 21.

The display 23, which includes a monitor such as an LCD, displays a variety of windows according to a display signal input from the hardware processor 21. For example, the display 23 displays a variety of web pages based on display data for displaying the variety of web pages received from the data management apparatus 10.

The communication section 24, which is constituted by a network interface and the like, sends and receives data to and from an external device that is connected through the communication network N such as a LAN, a WAN or the Internet. Further, the communication section 24 may perform wireless communication by using a mobile network or the like to send and receive data to and from an external device such as the data management apparatus 10 that is connected to through the communication network N.

The RAM 25 forms a work area for temporality storing a variety of programs read out from the storage 26, input and output data and parameters in a variety of processing that is performed and controlled by the hardware processor 21.

The storage 26 is constituted by an HDD, a non-volatile semiconductor memory or the like. In the storage 26, a variety of processing programs, and parameters and files necessary for executing the programs are stored. For example, a web browser program for providing a function as a web browser and the like are stored in the storage 26.

### OPERATION IN HOME CARE COOPERATION SYSTEM

Next, the operation in the home care cooperation system 100 will be described.

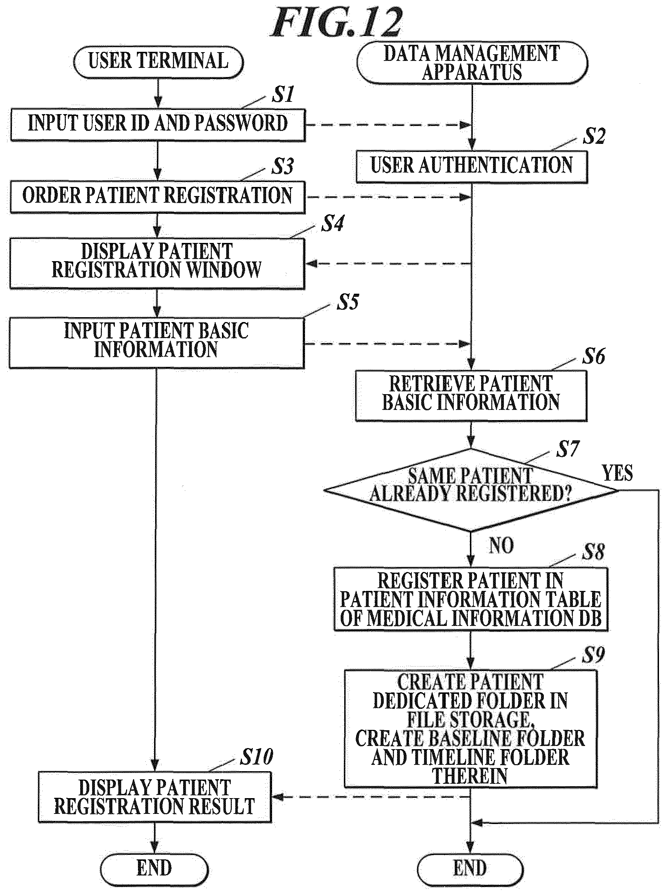

FIG. 12 is a ladder chart of patient registration processing that is performed by the data management apparatus 10 and a user terminal 20 of the home care cooperation system 100. This processing is performed to register a patient who is cared at home. The processing in the data management apparatus 10 is achieved by software processing of the hardware processor 11 in cooperation with a program stored in the storage 16, and the processing in the user terminal 20 is achieved by software processing of the hardware processor 21 in cooperation with a program stored in the storage 26.

First, in the user terminal 20, in response to an input of a URL for accessing to the data management apparatus 10 through a web browser on the operation section 22, the input URL is sent to the data management apparatus 10 through the communication section 24.

In the data management apparatus 10, in response to a reception of the URL for accessing to the data management apparatus 10 from the user terminal 20 through the communication section 14, a display data for displaying a login window is sent to the user terminal 20. The display data for displaying a variety of web pages including the login window, which is sent to the user terminal 20 by means of a web server function of the data management apparatus 10, includes HTML data, a style sheet, image data, a script for making the user terminal 20 perform predetermined processing, and the like.

In the user terminal 20, in response to a reception of the display data for the login window through the communication section 24, the login window is displayed on the display 23. The login window includes input boxes for a user ID and a password. In the login window, in response to an operation on the operation section 22 of inputting a user ID and a password, the input user ID and the password are sent to the data management apparatus 10 through the communication section 24 (Step S1).

In the data management apparatus 10, in response to a reception of the user ID and the password through the communication section 14, user authentication is performed (Step S2). Specifically, the user management table T1 as illustrated in FIG. 3 is searched for a record that matches the received user ID. When the password of the record matches the received password, it is determined that the user authentication succeeded. When the user authentication succeeded, the home care cooperation service is provided according to an operation on the web browser of the user terminal 20. When the received user ID is not found in the user management table T1 or the received password does not match the password of the retrieved record, it is determined that the user authentication failed.

Next, in the user terminal 20, in response to an operation on the operation section 22 of registering a patient, a patient registration order is sent to the data management apparatus 10 through the communication section 24 (Step S3).

In the data management apparatus 10, in response to a reception of the patient registration order from the user terminal 20 through the communication section 14, display data for displaying a patient registration window is sent to the user terminal 20.

In the user terminal 20, in response to a reception of the display data for the patient registration window through the communication section 24, the patient registration window is displayed on the display 23 (Step S4). The patient registration window includes input boxes for patient basic information (name, gender, birth date, address, telephone number, etc. of a patient). In the patient registration window, in response to an operation on the operation section 22 of inputting patient basic information of a patient to be registered, the input patient basic information is sent to the data management apparatus 10 through the communication section 24 (Step S5).

In the data management apparatus 10, in response to an acquisition of the patient basic information through the communication section 14 (Step S6), the hardware processor 11 references the basic information table T21 of the medical information DB 161 to make a determination as to whether the same patient is already registered (Step S7).

When the same patient is already registered (Step S7, Yes), the patient registration processing ends.

In Step S7, when the patient is not registered yet (Step S7, No), the hardware processor 11 registers the patient in the patient information table T2 of the medical information DB 161 (Step S8). Specifically, the hardware processor 11 adds a new record to the patient information table T2 and stores a newly created patient ID therein. Then, the hardware processor 11 creates a basic information table T21, a baseline table T22, a timeline table T23 and an access restriction table T24 of the newly registered patient, and stores information indicating links to the tables T21 to T24 in the patient information table T2 along with the patient ID. Further, the patient basic information input from the user terminal 20 is stored in the basic information table T21 of the newly registered patient.

The access restriction defined in the access restriction table T24 may be set either during the patient registration processing or after the patient registration processing by another medical personal or the like.

Then, in the data management apparatus 10, the hardware processor 11 creates a dedicated patient folder for the newly registered patient in the file storage 162 and further creates a baseline folder and a timeline folder in the dedicated patient folder (Step S9).

Then, display data for displaying a patient registration result (succeeded or failed) is sent to the user terminal 20 through the communication section 14.

In the user terminal 20, in response to a reception of the display data for the patient registration result through the communication section 24, the patient registration result is displayed on the display 23 (Step S10).

Then, the patient registration processing ends.

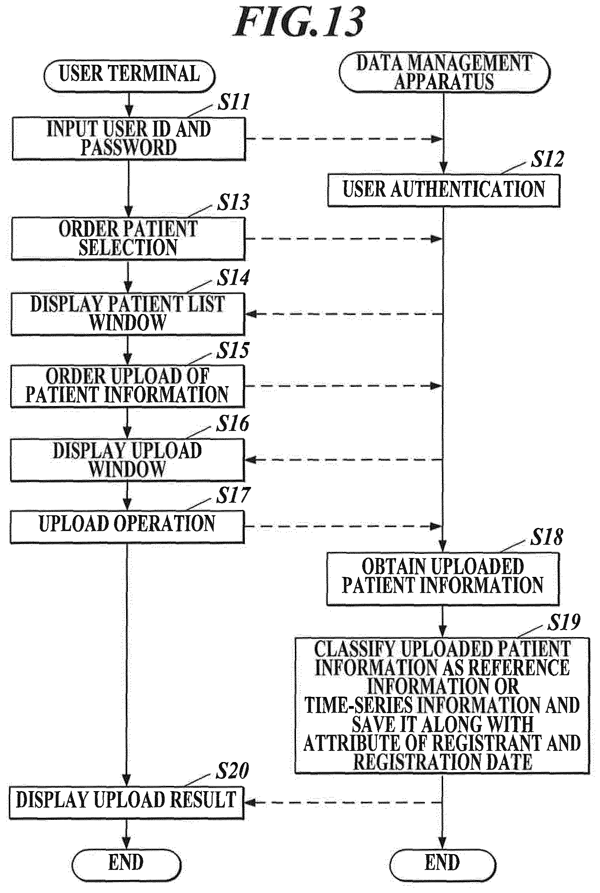

FIG. 13 is a ladder chart of patient information upload processing that is performed by the data management apparatus 10 and a user terminal 20 of the home care cooperation system 100. This processing is performed to register patient information. The processing in the data management apparatus 10 is achieved by software processing of the hardware processor 11 in cooperation with a program stored in the storage 16, and the processing in the user terminal 20 is achieved by software processing of the hardware processing 21 in cooperation with a program stored in the storage 26.

For example, when a patient is discharged from a hospital, a letter of introduction, a discharge summary and the like are uploaded. After starting the home care, new information is daily generated as a result of a home visit, nursing, care and the like, which is uploaded and accumulated.

The processing in Step S11 and Step S12 is the same as that in Step S1 and Step S2 in FIG. 12, and the description thereof is omitted.

Then, in the user terminal 20, in response to an operation on the operation section 22 of selecting a patient, a patient selection order is sent to the data management apparatus 10 through the communication section 24 (Step S13).

In the data management apparatus 10, in response to a reception of the patient selection order from the user terminal 20 through the communication section 14, display data for displaying a patient list window is sent to the user terminal 20.

In the user terminal 20, in response to a reception of the display data for the patient list window through the communication section 24, the patient list window is displayed on the display 23 (Step S14).

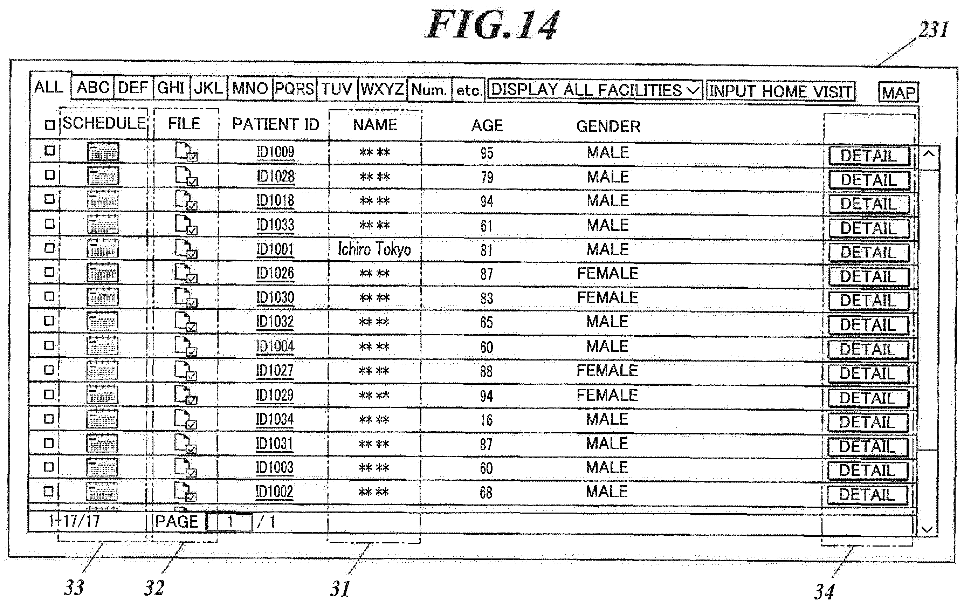

FIG. 14 illustrates an example of the patient list window 231. The patient list window 231 includes a list of patients in which information on each patient is displayed in single line. In the patient list window 231, a patient name 31, a file icon 32, a schedule icon 33, a detail button 34 and the like are displayed with respect to each patient.

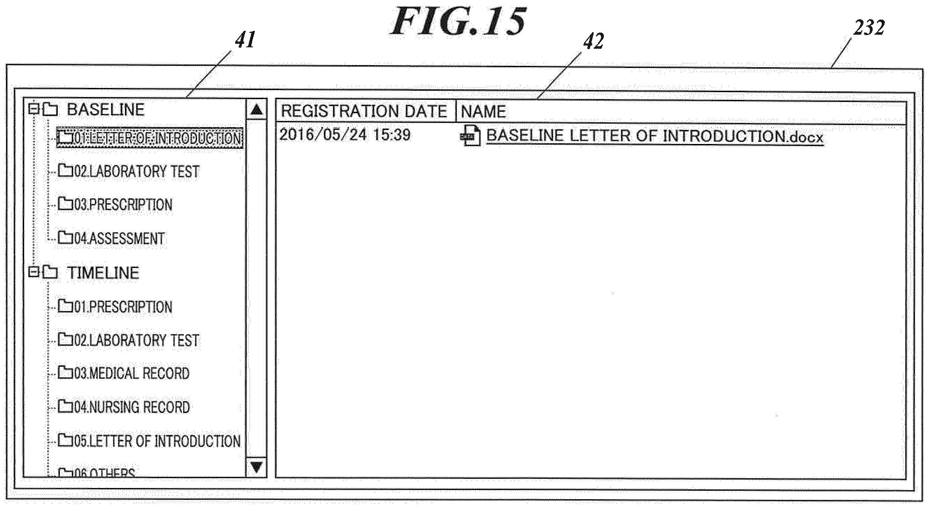

In response to an operation on the operation section 22 of a click on a file icon 32, a file management window 232 (see FIG. 15) of the patient corresponding to the clicked file icon 32 is displayed.

In response to an operation on the operation section 22 of a click on a schedule icon 33, a window for managing the schedule related to the patient corresponding to the clicked schedule icon 33 is displayed.

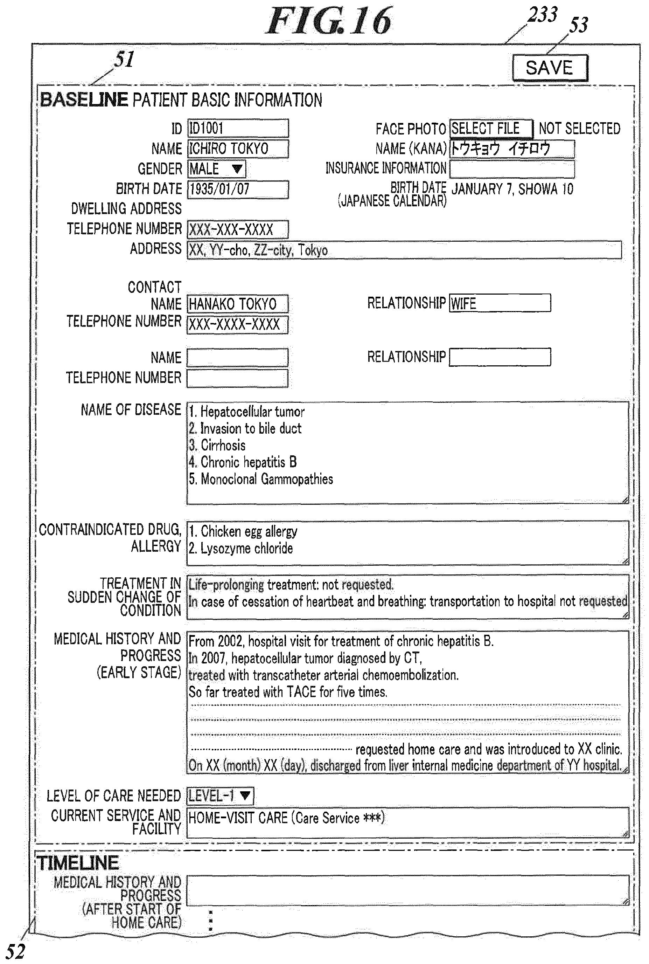

In response to an operation on the operation section 22 of a click on a detail button 34, an edit window 233 (see FIG. 16) for inputting character/number information of patient information of the patient corresponding to the clicked detail button 34 is displayed.

The patient who corresponds to the clicked file icon 32 or detail button 34 in the patient list window 231 is set as a target patient whose patient information is to be registered.

Then, in the user terminal 20, in response to an operation on the operation section 22 of an order to upload the patient information, a patient information upload order is sent to the data management apparatus 10 through the communication section 24 (Step S15).

In the data management apparatus 10, in response to a reception of the patient information upload order from the user terminal 20 through the communication section 14, display data for displaying an upload window is sent to the user terminal 20.

In the user terminal 20, in response to a reception of the display data for the upload window through the communication section 24, the upload window is displayed on the display 23 (Step S16).

When the information to be registered is file information, a file management window 232 as illustrated in FIG. 15 is displayed as the upload window. The file management window 232 incudes a folder display area 41 and a file selection area 42.

In the folder display area 41, the folder structure in the dedicated patient folder of the target patient in the file storage 162 is displayed. In response to an operation on the operation section 22 of a left click of a mouse with the mouse pointer over a folder, the clicked folder is selected. In response to an operation on the operation section 22 of a right click of a mouse with the mouse pointer over a folder, a menu is displayed. A new folder can be created by clicking the right button of a mouse over a baseline folder or a timeline folder and selecting an item in the displayed menu.

By an operation on the operation section 22 of dragging a file to be uploaded from among files displayed on another window and dropping it in the file selection area 42 on the display 23, the dropped file is uploaded to the folder that is selected in the folder display area 41. Based on the folder selected in the folder display area 41, a determination is made whether the file to be uploaded is classified as reference information or time-series information.

When the information to be registered is character/number information, an edit window 233 as illustrated in FIG. 16 is displayed as the upload window. The edit window 233 includes input boxes for items of patient information. The edit window 233 includes a baseline input area 51 and a timeline input area 52, and information is classified as reference information or time-series information based on the input area to which the information is input. By inputting information in a corresponding input box and pressing a save button 53, the input information is uploaded to the data management apparatus 10.

In the user terminal 20, in response to an operation on the operation section 22 of an upload operation on the upload window (Step S17), the patient information to be uploaded is sent to the data management apparatus 10 through the communication section 24. When the patient information is file information, the upload operation corresponds to a file drop operation on the file management window 232. When the patient information is character/number information, the upload operation corresponds to inputting information in input boxes and pressing the save button 53 in the edit window 233.

In the data management apparatus 10, in response to an acquisition of the patient information to be uploaded through the communication section 14 (Step S18), the hardware processor 11 classifies the patient information to be uploaded as reference information or time-series information and saves it along with the attribute of the registrant and the registration date (Step S19). Specifically, a baseline table T22 or a timeline table T23 that is linked to the patient ID of the target patient is specified in the patient information table T2 of the medical information DB 161 based on the classification of the uploaded patient information, and the patient information (item name and content, or item name and file path) is stored in the specified baseline table T22 or timeline table T23 as well as the user ID of the registrant and the registration date. Further, the attribute that corresponds to the user ID of the user who is currently using (logged in) the home care cooperation system 100 is retrieved from the user management table T1 of the medical information DB 161 and is stored as the registrant attribute in the baseline table T22 or the timeline table T23.

When the patient information is file information, it is saved in the file storage 162 in a folder that was selected in the file management window 232 (at the storage location indicated by the file path).

Then, in the data management apparatus 10, display data for displaying the upload result (succeeded or failed) is sent to the user terminal 20 through the communication section 14.

In the user terminal 20, in response to a reception of the display data for the upload result through the communication section 24, the upload result is displayed on the display 23 (Step S20).

Then, the patient information upload processing ends.

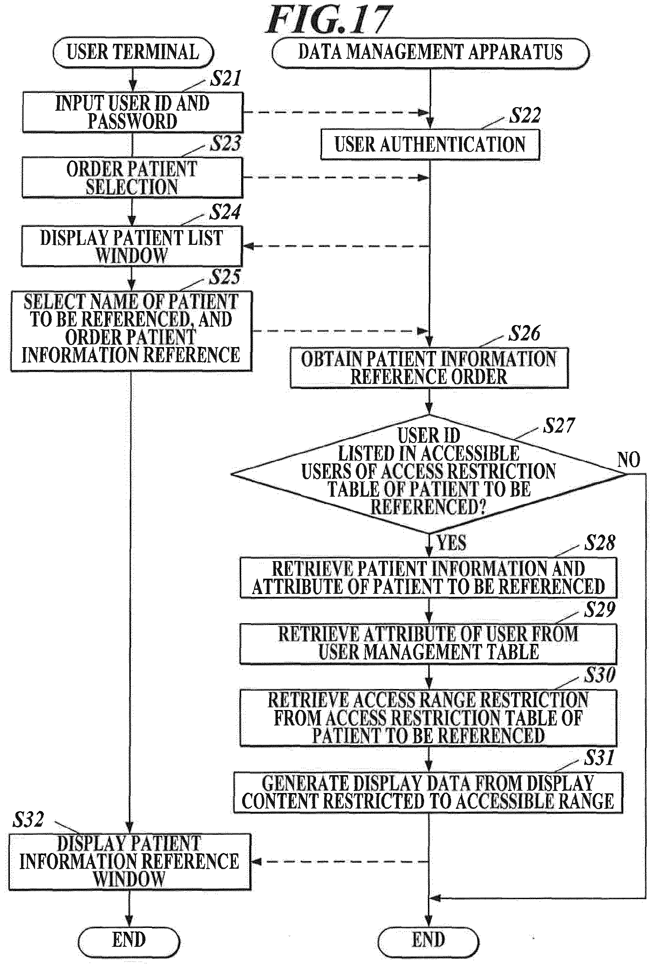

FIG. 17 illustrates a ladder chart of patient information reference processing that is performed by the data management apparatus 10 and a user terminal 20 of the home care cooperation system 100. This processing is performed to reference patient information. The processing in the data management apparatus 10 is achieved by software processing of the hardware processor 11 in cooperation with a program stored in the storage 16, and the processing in the user terminal 20 is achieved by software processing of the hardware processing 21 in cooperation with a program stored in the storage 26.

The processing in Step S21 to Step S24 is the same as that in Step S11 to Step S14 in FIG. 13, and the description thereof is omitted.

Then, in the user terminal 20, in response to an operation on the operation section 22 of selecting the name of a patient to be referenced in the patient list window and making an order to reference the patient information, a patient information reference order is sent to the data management apparatus 10 through the communication section 24 (Step S25).

For example, when any one of the patient names 31 is selected in the patient list window 231 as illustrated in FIG. 14, a patient information reference order corresponding to the selected patient name 31 is sent to the data management apparatus 10.

In the data management apparatus 10, in response to an acquisition of the patient information reference order through the communication section 14 (Step S26), the hardware processor 11 references the patient information table T2 of the medical information DB 161 to specify the access restriction table T24 that is linked to the patient ID of the patient to be referenced. Then, the hardware processor 11 references the access restriction table T24 of the patient to be referenced and makes a determination as to whether the user ID of the currently logged-in user is included in those of the users with access permission (Step S27).

When the user ID of the currently logged-in user is not included in the users with access permission (Step S27, No), the patient information reference processing ends.

In Step S27, when the user ID of the currently logged-in user is included in the users with access permission in the access restriction table T24 of the patient to be referenced (Step S27, Yes), the hardware processor 11 references the patient information table T2 of the medical information DB 161 to specify a baseline table T22 and a timeline table T23 that are linked to the patient ID of the patient to be referenced. Then, the hardware processor 11 references the specified baseline table T22 and the timeline table T23 to retrieve the patient information (reference information and time-series information) of the patient to be referenced and the attribute of the user who registered the patient information (Step S28). Specifically, when the patient information is character/number information, the item name, the content and the attribute of the registrant are retrieved from the baseline table T22 or the timeline table T23. When the patient information is file information, the item name, the file path and the attribute of the registrant are retrieved from the baseline table T22 or the timeline table T23, and the corresponding file is retrieved from the file storage 162 according to the retrieved file path. When the patient information is time-series information, the registration date is also retrieved from the timeline table T23.

Then, the hardware processor 11 retrieves the attribute corresponding to the user ID of the currently logged-in user from the user management table T1 of the medical information DB 161 (Step S29).

Then, the hardware processor 11 references the access restriction table T24 of the patient to be referenced to retrieve the accessible range restriction for users with the attribute of the currently logged-in user (Step S30). Specifically, information is retrieved from the "patient/family" field, the "nursing/care" field or the "medical care" field corresponding to the attribute of the currently logged-in user in the access restriction table T24 of the patient to be referenced.

Then, the hardware processor 11 generates display data for displaying a patient information reference window in which the contents to be displayed of the patient information retrieved in Step S28 are restricted to the accessible range of the currently logged-in user based on the retrieved accessible range restriction (Step S31). Then, the display data thus generated is sent to the user terminal 20 through the communication section 14.

In the user terminal 20, in response to a reception of the display data for the patient information reference window through the communication section 24, the patient information reference window is displayed on the display 23 (Step S32).

Then, the patient information reference processing ends.

FIG. 18 illustrates an example of the patient information reference window 234 that is displayed on the display 23 of the user terminal 20. The patient information reference window 234 includes a patient identification information display area 61 and a patient information display area 62.

In the patient identification information display area 61, information for identifying the patient is always displayed.

The patient information display area 62 is divided into three areas of the upper, middle and lower columns, which are respectively allocated as a patient/family area 63, a nursing/care area 64, and a medical care area 65. Further, the patient information display area 62 is divided into left and right areas, which are respectively allocated as a baseline display area 66 and a timeline display area 67.

In the patient/family area 63, patient information that was registered by users belonging to the "patient/family" is displayed.

In the nursing/care area 64, patient information that was registered by users belonging to the "nursing/care" is displayed.

In the medical care area 65, patient information that was registered by the users belonging to the "medical care" is displayed.

When there is an access restriction, information that is not permitted to be accessed is not displayed according to the attribute of the currently logged-in user.

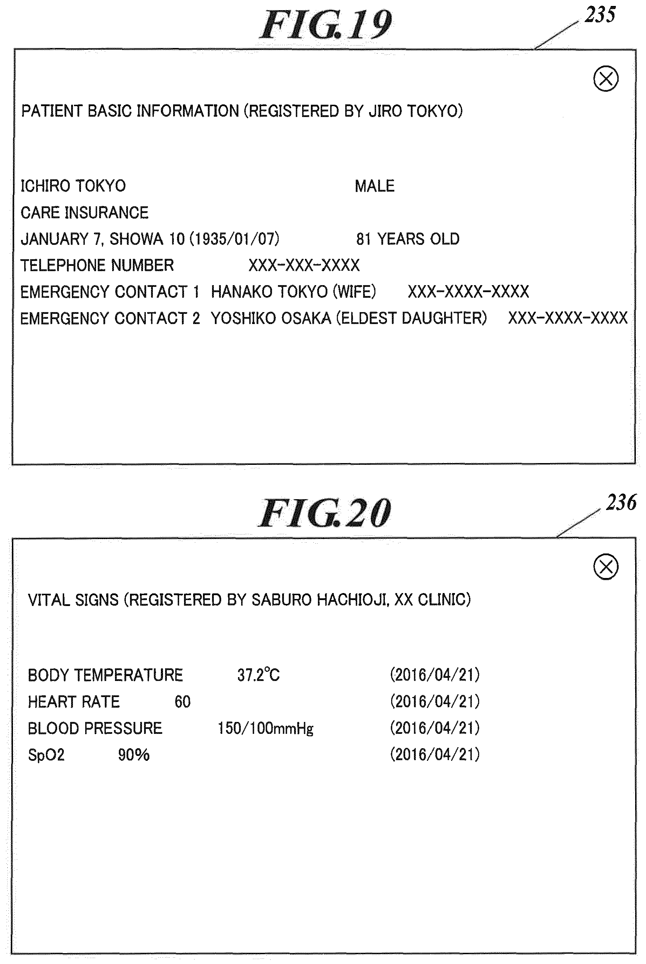

In the baseline display area 66, items of the reference information at the time when a patient started to be cared at home is displayed. In response to an operation on the operation section 22 of a click on any one of the item names, the hardware processor 11 of the data management apparatus 10 generates display data for displaying the content corresponding to the clicked item name in another window. For example, when an item name 68 "patient basic information" is clicked, which is in the area where the baseline display area 66 and the patient/family area 63 overlap each other, a patient basic information window 235 as illustrated in FIG. 19 is displayed on the display 23. In the patient basic information window 235, patient basic information that was registered by users with an attribute of "patient/family" is displayed.

Since the baseline display area 66 is further divided into the patient/family area 63, the nursing/care area 64 and the medical care area 65, it is easy to understand which information was registered by a user with a certain attribute.

In the timeline display area 67, the presence or absence of information is displayed at corresponding dates on the timeline with respect to each item of the time-series information that is daily generated as a result of home care, nursing and care. The time window and the start date of the displayed timeline can be changed.

Specifically, a mark is displayed with respect to each item at the position corresponding to the date when the item of information was registered. In response to an operation on the operation section 22 of a click on any one of such marks, the hardware processor 11 of the data management apparatus 10 generates display data for displaying the information that was registered at the date corresponding to the position of the mark. For example, when a mark 69 on April 21 in the item "vital signs" is clicked, which is in the area where the timeline display area 67 and the medical care area 65 overlap each other, a vital sign window 236 as illustrated in FIG. 20 is displayed on the display 23. In the vital sign window 236, vital signs that were registered by users with an attribute of "medical care" are displayed.

Alternatively, such information may be automatically displayed when the mouse pointer is placed over a mark.

Also regarding the timeline display area 67, since it is further divided into the patient/family area 63, the nursing/care area 64 and the medical care area 65, it is easy to understand which information was registered at a certain date by a user with a certain attribute.

The display size of the patient/family area 63, the nursing/care area 64 and the medical care area 65 is adjustable by an operation on the operation section 22 of vertically shifting the boundary between the patient/family area 63 and the nursing/care area 64 or the boundary between the nursing/care area 64 and the medical care area 65.

The display size of the baseline display area 66 and the timeline display area 67 is adjustable by an operation on the operation section 22 of horizontally shifting the boundary between the baseline display area 66 and the timeline display area 67.

In response to an operation on the user terminal 20 of changing a boundary position of the areas, the coordinate of the changed boundary (e.g. the coordinates of the upper-left and lower-right corners of the area) is sent to the data management apparatus 10 through the communication section 24.

In the data management apparatus 10, the hardware processor 11 saves the coordinate of the changed boundary in the user management table T1 of the medical information DB 161 as a display parameter along with the user ID of the user who made the adjustment.

After the adjustment, when the same user uses the home care cooperation system 100, the areas in the patient information reference window are displayed in the saved arrangement according to the display parameter stored in the user management table T1.

As described above, in the home care cooperation system 100 according to the embodiment, patient information is displayed in a single screen in a classified manner as reference information or time-series information, and the respective areas are further divided according to attributes. This enables displaying patient information in an easy-to-understand manner while indicating the classification of the patient information and the source of each item of the information.

For example, since the reference information and the time-series information are displayed in a single screen, it is possible to understand each information in a short time without screen transition between the reference information and the time-series information.

Further, the reference information and the time-series information are further classified according to the attribute of the registrant. For example, since information registered by another physician is displayed in the same area as information registered by a primary care physician, it is easy to reference patient information.

Since information at the start of home care and information generated after the start of the home care are displayed in divided respective areas, it is easy to understand the history of the patient information of a home care patient.

By changing the display size of the area in which the reference information is displayed and the area in which the time-series information is displayed in a single screen or changing the display size of the areas for the respective attributes in the single screen, it is possible to change the display manner to be easier to understand for a user who references the patient information.

The contents to be displayed can be restricted according to the attribute of the user who currently uses the system.

The above description of the embodiment merely illustrates an example of the patient information display system according to the present invention, and the present invention is not limited thereto. Suitable changes can be made in the detailed configuration and the detailed operation of the apparatuses of the system without departing from the features of the present invention.

For example, the above-described embodiment is an example in which the predetermined time point corresponds to the time point when a patient started to be cared at home. However, the predetermined time point is not limited thereto.

In the patient information reference window, the direction of dividing the window according to the attributes and the direction of dividing the window into reference information and time-series information are not limited to the illustrated example.

Further, the above-described embodiment is an example in which the patient information reference processing is configured such that item names or marks indicating the presence or absence of information are displayed first when the reference information and the time-series information of a patient to be referenced are displayed on a user terminal 20, and the contents thereof is displayed in another window. However, the contents of the reference information and the time-series information may be directly displayed in the patient information reference window.

The patient information reference processing may also be configured such that only the latest information of each item is displayed in the timeline display area 67 of a user terminal 20 from among the time-series information of a target patient. In this configuration, the hardware processor 11 of the data management apparatus 10 generates display data for displaying only the latest information of each item on the display 23 of the user terminal 20 from among the time-series information of the target patient. Specifically, regarding the time-series information of the target patient, the hardware processor 11 specifies the timeline table T23 that is linked to the patient ID of the target patient in the patient information table T2 and references the specified timeline table T23 to extract only the record on the latest registration date with respect to each item. Then, based on the extracted records, the hardware processor 11 lays out the latest information of each item in the display area for the time-series information to generate the display data. Therefore, it is possible to readily understand the latest information of the patient.

## Claims

1. A patient information display system (100), comprising:
a storage (16) for storing patient information on a patient in a classified manner as reference information which indicates a condition of the patient at a predetermined time point or time-series information which is generated after the predetermined time point and is accumulated as time proceeds,
wherein each of the patient information is stored in the storage (16) along with an attribute of a user who registered the patient information,
wherein the patient information display system (100) further comprises: a hardware processor (11) which retrieves patient information of a target patient and an attribute linked to the patient information from the storage (16) and displays the patient information of the target patient in a single screen on a display (13) in which the screen is divided into respective areas for reference information and time-series information with respect to each attribute.

2. The patient information display system (100) according to claim 1, wherein the predetermined time point corresponds to a time point when home care was started.

3. The patient information display system (100) according to claim 1 or 2, wherein the attribute includes patient/patient family, nursing/care, and medical care.

4. The patient information display system (100) according to any one of claims 1 to 3, further comprising a changer (11) which changes a display size of the respective areas for displaying the reference information and the time-series information in the single screen.

5. The patient information display system (100) according to any one of claims 1 to 4, further comprising a second changer (11) which changes a display size of areas for respective attributes in the single screen.

6. The patient information display system (100) according to any one of claims 1 to 5, further comprising:
a second storage (16) for storing users along with an attribute of each user; and
a third storage (16) for storing information indicating accessibility to patient information of each patient with respect to each attribute of the users,
wherein the hardware processor (11) retrieves an attribute of a user who currently uses the patient information display system from the second storage (16) and retrieves information indicating accessibility to the patient information of the target patient for users with the retrieved attribute from the third storage (16), so as to restrict content to be displayed on the display (13) based on the retrieved information indicating the accessibility.

7. The patient information display system (100) according to any one of claims 1 to 6, wherein the hardware processor (11) displays presence or absence of the time-series information of the target patient along a timeline.

8. The patient information display system (100) according to any one of claims 1 to 6, wherein the hardware processor (11) displays only latest information of each item from among the time-series information of the target patient.

9. A patient information display method for a patient information display system (100) that comprises a storage (16) for storing patient information on a patient in a classified manner as reference information which indicates a condition of the patient at a predetermined time point or time-series information which is generated after the predetermined time point and is accumulated as time proceeds,
wherein each of the patient information is stored in the storage (16) along with an attribute of a user who registered the patient information,
the method comprising:
retrieving patient information of a target patient and an attribute linked to the patient information from the storage (16); and
displaying the patient information of the target patient in a single screen on the display (13) in which the screen is divided into respective areas for reference information and time-series information with respect to each attribute.
